# EUROPEAN PATENT APPLICATION

(11) **EP 2 944 349 A1**
(43) Date of publication of application: **18.11.2015**
(21) Application number: 15167816.6
(22) Date of filing: 15.05.2015
(51) Int. Cl.: A61M 37/00

(54) **ADJUSTABLE GRIP FOR A TATTOO MACHINE**

(30) Priority: 15.05.2014 GB 201408682
(71) Applicant: EGO Tubes Limited, Liverpool L8 7BA (GB)
(72) Inventor: Beston, Richard, Sunderland, Tyne and Wear SR4 8RP (GB)
(74) Representative: Hancox, Jonathan Christopher

(57) **Abstract**

Adjustable grip for a tattoo machine 50 comprising a handgrip portion 20 for the end user to hold during use and adapted to receive a needle cartridge 10, a backstem 40 adapted to engage with the oscillating bar of a tattoo machine 50, an annular clamp portion 25 and a movable collar 30. The collar 30 is movable between a first position in which the collar engages with the annular clamp portion 25 causing the annular clamp portion to grip the backstem 40, and a second position which allows the annular clamp portion 25 to release the backstem 40 so that the position of the handgrip portion 20 relative to the backstem 40 can be adjusted. Moving the grip backwards and forwards over the backstem 40 moves the needles 11 relative to the cartridge body.

## Description

Tattoo machines are hand-held devices used to create permanent marking of the skin (a tattoo) with indelible ink.

The most common method of tattooing involves inserting ink into the skin via a single needle, or a group of needles, which are attached to and driven by a bar, which is itself attached to an oscillating unit. The unit rapidly and repeatedly drives the needles in and out of the skin, usually 80 to 150 times a second.

Tattoo machines most commonly come in two types: "Coiled" machines that use electromagnetic coils to move the oscillating unit backwards and forwards, and "Rotary" machines which are powered by regulated motors rather than electromagnetic coils.

The bar oscillates back and forth inside a hollow tube known as a backstem. Attached to the outside of backstem is the grip, which is held by the end user (tattoo artist) during use. At the distal end of the backstem is the needle. Traditionally, needles were soldered directly onto the oscillating bar. However, some currently available tattoo machines, such as the Cheyenne^{®} Hawk, are specifically designed to utilise removable needle cartridges. The needle cartridge is inserted directly into the grip at the distal end. The cartridges allow rapid changing of needle configurations during the work process.

The cartridges comprise a needle cartridge body, which has "plunger" at one end. When the plunger is pushed in towards the cartridge body, it causes the needles at the other end to protract out of the cartridge body, "unsheathing" them. The oscillating bar engages with the end of the plunger inside the backstem and moves the needles back and forth.

Modern tattooing procedure is typically very sanitary. The needles (or cartridges) are single-use and come in individual sterilised packaging. The tattoo artist must wash his or her hands and must also wash the area that will be tattooed. Gloves must be worn at all times and the wound must be wiped frequently with a wet disposable towel of some kind. The equipment must be sterilized in a certified autoclave before every use.

During use, an artist is required to adjust how exactly far the needles will protract out of the cartridge when the machine is running, thus determining how deep the needles will penetrate the skin. This determines all different aspects and visuals of the tattoo, for instance shading, solid colouring, lining etc.

In more traditional machines, the grip that holds the cartridges is integral with the backstem which is locked in to a vice clamp. The back stem is locked in a vice clamp atop the tattoo machine. To adjust how far the needles will protract or retract is done by unscrewing the vice clamp, and moving the back stem (along with integral grip) and re-clamping in place using the vice clamp on the tattoo machine.

The nearer to the tattoo machine the backstem and grip are moved, the further out the needles will protrude from their cartridge during use.

More modern machines allow the grip and backstem to be attached by screw attachment to the machine. If the tattoo artist wants to adjust the needle depth, they need to simply twist the main body of the grip clockwise or anticlockwise, depending on whether they want to protract or retract the needles. This mechanism uses a ratchet like system and the grip is made up from many different parts. The problem with this grip, is it is not recommended to be autoclaved, and so it can be unhygienic as it is used on many different clients. The artist only has the option to wipe down the grip manually using disinfectants and putting a plastic shield around the grip before they hold it.

The present invention provides an improved mechanism for adjusting the needle depth. The needle length may be adjusted easily during use of the machine, and the simplicity of the mechanism means that the grip can be manufactured and sold at a low enough cost that is viable for a tattooist to deem as disposable and one use only, which has obvious hygiene benefits.

The present invention provides an adjustable grip for a tattoo machine comprising a handgrip portion for the end user to hold during use and adapted to receive a needle cartridge, a backstem portion adapted to engage with the oscillating bar of a tattoo machine, an annular clamp portion and a movable collar. The collar is movable between a first position in which the collar engages with the annular clamp portion causing the annular clamp portion to grip the backstem, and a second position which allows the annular clamp portion to release the backstem so that the position of the handgrip portion relative to the backstem can be adjusted.

Preferably, the backstem portion passes through the annular clamp, the movable collar and the handgrip portion.

Preferably the handgrip portion and annular clamp are fixed together.

Preferably the movable collar engages with the annular clamp in such a way that the collar surrounds the clamp, forcing it to close. When the clamp is closed around the backstem, it is fixed in place. As the collar is disengaged from the annular clamp, the clamp relaxes and opens, allowing the clamp (and associated handgrip and cartridge) to be relocated along the backstem.

Preferably the movable collar is threaded.

Preferably the movable collar is a nut.

Preferably either the handgrip portion or the annular clamp is threaded to engage with the thread of the collar. Screwing and unscrewing the collar therefore allows the handgrip portion to be relocated up or down the backstem and re-secured in place.

Moving the grip backwards and forwards over the backstem moves the needles relative to the cartridge body. The backstem remains stationary with respect to the tattoo machine, unlike traditional machines where the entire backstem and grip portion are moved together. The present invention allows tattoo artists to operate effectively with an improved adjustment mechanism. The device is so simple and cheap to manufacture that it may be treated as disposable, improving hygiene.

The present invention may be used with a rotary tattoo machine and may be used with needle cartridges.

Preferably the present invention utilises a screw-on attachment for the backstem to attach it to the tattoo machine.

The present invention also relates to a tattoo machine comprising an adjustable grip as described above.

An embodiment of the invention will now be described by way of example only and with reference to the accompanying drawings, in which:

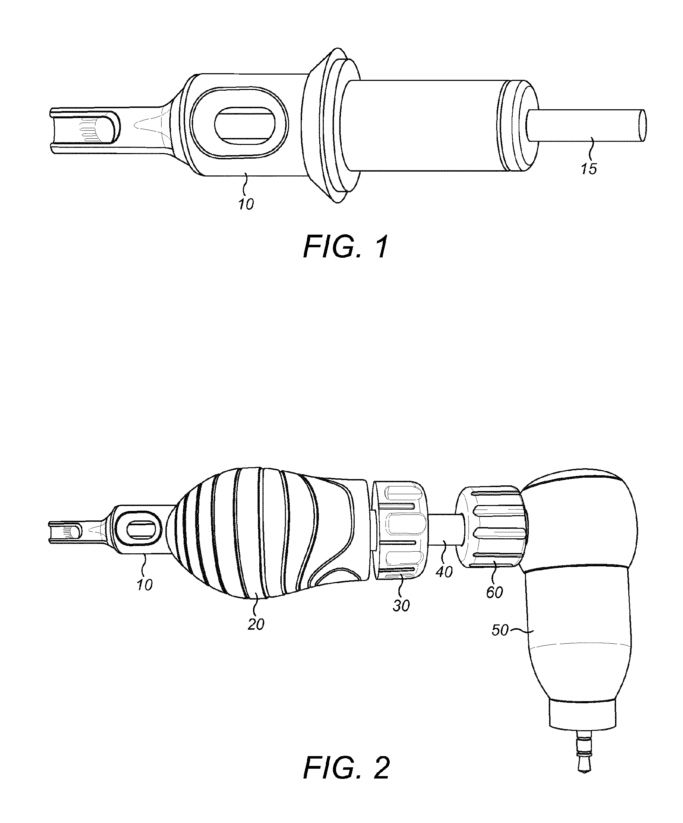

Figure 1 shows a detachable needle cartridge;
Figure 2 shows a tattoo machine including an adjustable grip in accordance with the invention in a first position.; and

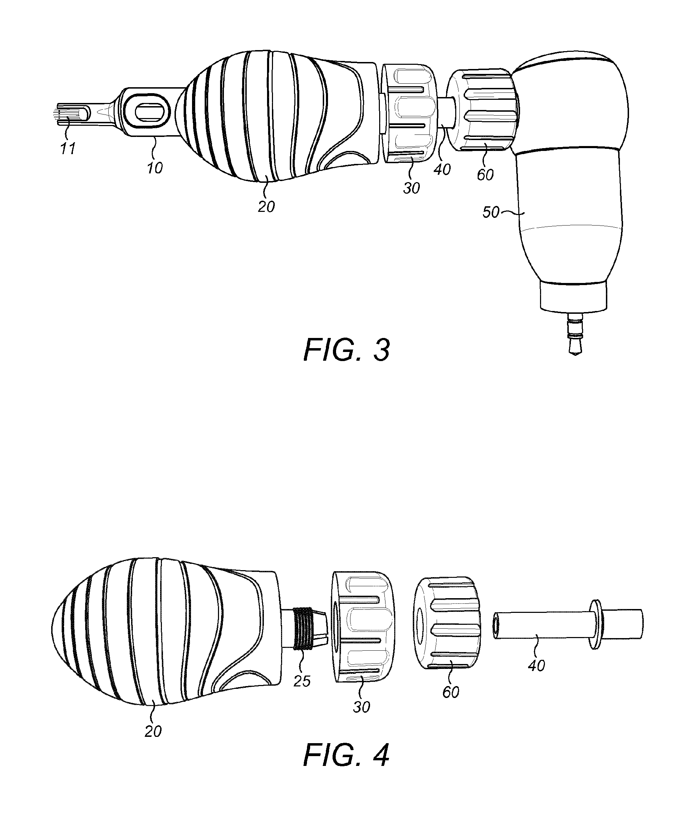

Figure 3 shows the tattoo machine of figure 2 in which the handgrip portion has been relocated nearer the tattoo machine.

The figures show the following features:
- 10: needle cartridge
- 11: needles
- 15: plunger
- 20: handgrip portion
- 25: annular clamp
- 30: movable collar
- 40: backstem
- 50: tattoo machine
- 60: screw-on attachment

With reference to the figures, the invention provides an adjustable grip for a tattoo machine 50 comprising a handgrip portion 20 for the end user to hold during use and adapted to receive a needle cartridge 10, a backstem 40 adapted to engage with the oscillating bar of a tattoo machine 50, an annular clamp portion 25 and a movable collar 30. Screwing and unscrewing the collar causes the annular clamp portion 25 to grip or release the backstem 40 so that the handgrip portion can be relocated up or down the backstem 40 and re-secured in place. Moving the grip backwards and forwards over the backstem 40 moves the needles 11 relative to the cartridge body and determines how far the needles 11 will protract out of the cartridge 10 when the machine is running.

In the present invention, the backstem remains stationary with respect to the tattoo machine, unlike traditional machines where the entire backstem and grip portion are moved together. The present invention allows tattoo artists to operate effectively with an improved adjustment mechanism. The device is simple and cheap to manufacture so that it may be treated as disposable, improving hygiene.

## Claims

1. An adjustable grip for a tattoo machine (50) comprising a handgrip portion (20) for the end user to hold during use and adapted to receive a needle cartridge (10), a backstem (40) adapted to engage with an oscillating bar of the tattoo machine (50), an annular clamp portion (25) and a movable collar (30), wherein the collar (30) is movable between a first position in which the collar engages with the annular clamp portion (25) causing the annular clamp portion to grip the backstem (40), and a second position which allows the annular clamp portion (25) to release the backstem (40) so that the position of the handgrip portion (20) relative to the backstem (40) can be adjusted.

2. The adjustable grip of claim 1, wherein the backstem (40) passes through the annular clamp (25), the movable collar (30) and the handgrip portion (20).

3. The adjustable grip of claim 1 or 2, wherein the handgrip portion (20) and annular clamp (25) are fixed together.

4. The adjustable grip of any preceding claim wherein, in the first position, the movable collar (30) engages with the annular clamp (25) by surrounding the clamp, forcing it to close onto the backstem (40) and fixing the backstem in place.

5. The adjustable grip of claim 4, wherein, in the second position, the movable collar (30) is disengaged from the annular clamp (25) allowing the clamp to relax and open, allowing the clamp (25) and handgrip portion (20) to be relocated along the backstem (40).

6. The adjustable grip of any preceding claim, wherein the movable collar (30) is threaded.

7. The adjustable grip of claim 6, wherein the annular clamp (25) is threaded to engage with the thread of the collar (30) such that screwing and unscrewing the collar allows the handgrip portion to be relocated up or down the backstem and re-secured in place.

8. The adjustable grip of any preceding claim, wherein the backstem (40) has a screw-on attachment (60) for attachment to the tattoo machine (50).

9. A tattoo machine (50) comprising an adjustable grip as claimed in any preceding claim.
